# FASCICULE DE BREVET EUROPEEN

(11) **EP 0 413 615 B1**
(45) Date de publication et mention de la délivrance du brevet: **24.05.1995**
(21) Numéro de dépôt: 90400558.4
(22) Date de dépôt: 28.02.1990
(51) Int. Cl.: A23K 1/00, C12N 1/04

(54) **Procédé de protection et de conservation de micro-organismes notamment de bactéries**
Verfahren zum Schützen und Konservieren von Mikroorganismen, insbesondere von Bakterien
Process for the protection and conservation of micro-organisms, particularly of bacteria

(30) Priorité: 17.07.1989 FR 8909583
(43) Date de publication de la demande: 20.02.1991
(73) Titulaire: Legarda, Joseph-Louis, F-33980 Audence (FR)
(72) Inventeur: Legarda, Joseph-Louis, F-33980 Audence (FR)
(74) Mandataire: Rodhain, Claude

(56) Documents cités:
- EP-A- 0 120 369
- EP-A- 0 131 114
- BE-A- 515 275
- BE-A- 820 786
- FR-A- 1 043 188
- FR-A- 2 242 037
- FR-A- 2 609 044
- CHEMICAL ABSTRACTS, vol. 103, no. 3, Juillet 1985, Columbus, Ohio,US; abrégéno. 21509H, G. JANOS: "Feed mixture containing protein-rich fodder plants,especially alfalfa, for monogastric animals", page 487,& HU-A-33 965 (G. JANOS et al.)

## Description

La présente invention a pour objet un procédé de protection et de conservation de micro-organismes notamment de bactéries.

Les micro-organismes comme les levures ou les bactéries sont aujourd'hui couramment utilisés dans le domaines de l'agronomie et de l'agro-alimentaire. Il est donc nécessaire de disposer de moyens efficaces permettant de conserver leurs activités biologiques et de les protéger contre les actions extérieures lors d'un stockage en vue de leur utilisation ultérieure. Actuellement les techniques de protection et de conservation les plus utilisées sont la congélation et la lyophilisation. Ces deux procédés donnent des résultats très satisfaisants, mais soumettent les bactéries à des variations de pression ou de température susceptibles de nuire à leur intégrité, et présentent en outre l'inconvénient de nécessiter un équipement coûteux.

Le brevet français N° 76/32722 décrit un procédé de protection et de conservation des micro-organismes avec des argiles, en vue d'application en agronomie pour le traitement des sols.

Cette technique n'est pas applicable dans le domaine agro-alimentaire où les bactéries doivent être incorporées a l'alimentation. En effet, les activités enzymatiques de certaines bactéries en font des additifs biologiques présentant une efficacité zootechnique particulièrement intéressante, notamment au niveau du tractus gastro-intestinal pour l'utilisation optimale de la ration alimentaire chez les animaux.

La demande de brevet européen EP-A-0 131 114 décrit un procédé de production d'une préparation sèche et stable, sous forme de poudre ou de granulés, contenant des bactéries revitalisables, qui après réhydratation contient au moins 10⁹ cellules viables par gramme de préparation, ce procédé comportant une étape de séchage de la masse produite au moyen d'un gaz très chaud.

La demande de brevet français FR-A-2 609 044 décrit une méthode pour la préparation d'une association de Lactobacillus bulgaricus et Streptococcus thermophilus dans laquelle les deux bactéries sont inoculées, en condition stérile, dans un terrain cultural à base de lait en poudre et/ou de sérum en poudre, d'extrait de malt, d'autolysat de levain et d'autres substrats culturaux appropriés.

La présente invention a donc pour but de proposer un procédé de protection et de conservation de bactéries peu onéreux, aisé et permettant la consommation des bactéries directement par les animaux.

Le procédé de l'invention consiste à effectuer au moins les trois étapes successives suivantes dans des conditions non stériles :
- une première étape de reproduction de bactéries consistant en un mélange de bactéries issues de collection et de bactéries obtenues par prélèvement dans la nature,
- une deuxième étape de tamponnage par mise en contact des bactéries, après l'étape de reproduction, avec un milieu nutritif contenant de la poudre de lait enrichie en biocatalyseur et minéraux,
- une troisième étape de stabilisation par mise en contact du produit obtenu à la seconde étape avec un milieu contenant au moins un extrait de céréales.

Les bactéries, après reproduction, sont ainsi déshydratées progressivement en présence d'un milieu nutritif.

Selon une forme particulière de mise en oeuvre de l'invention , l'étape de reproduction des bactéries est effectuée pendant une durée de 12 à 48 heures.

L'étape de reproduction permet l'obtention d'une population mixte de bactéries stables dans laquelle se trouvent toutes les bactéries importantes pour le mélange probiotique.

Ainsi, en fin de reproduction, on dispose d'un nombre de bactéries lactiques supérieur ou égal à 10⁷ par ml et un nombre de levures supérieur ou égal à 10⁴ par ml.

Selon une autre forme particulière de mise en oeuvre de l'inventions l'étape de tamponnage est réalisée par mise en contact des bactéries, après l'étape de reproduction, avec une poudre de lait enrichie en minéraux.

Parmi les minéraux préférés on peut citer les catalyseurs.
De manière avantageuse l'étape de tamponnage est effectuée pendant une durée de 18 à 24 heures.

L'étape de tamponnage assure une première absorption de l'eau contenue dans les bactéries, ce qui les préparent au choc osmotique auquel elles seront soumises à l'étape de stabilisation, tout en leur apportant les éléments nutritifs nécessaires à leur métabolisme.

Selon une forme préférée de mise en oeuvre de l'invention, l'étape de stabilisation est réalisée par mise en contact du produit obtenu à la deuxième étape avec au moins un extrait de céréale tels que le maïs, le blé, le soja, le riz ou un mélange de ceux-ci.

Avantageusement, l'étape de stabilisation est effectuée par des rafles de maïs broyées, extrudées ou floconnées, pendant une durée de 45 minutes.

Selon une forme avantageuse de mise en oeuvre de l'invention, les trois étapes sont réalisées dans des conditions non stériles.

L'invention sera bien comprise à la lecture de la description qui suit, donnée uniquement à titre d'illustration du procédé selon l'invention.

Les bactéries sont sélectionnées à partir de familles ou souches bactériennes dont les activités enzymatiques sont connues et choisies en fonction de l'utilisation biologique qui est envisagée.

Ces familles et souches bactériennes peuvent être issues de collection et donc être bien identifiées et répertoriées ou obtenues par des techniques classiques de prélèvement dans la nature, dilution, ensemencement et culture sur milieu nutritif.

On peut utiliser un mélange de bactéries constitué à la fois de bactéries issues de collections telles que :
- Lactobacillus plantarum
- Lactobacillus acidophilus
(ces souches sont des bactéries lactiques, hôtes habituels du tube digestif des mammifères),
et de bactérie obtenues par des prélèvements dans la nature lesquelles présentent souvent, suite à des mutations naturelles, une résistance accrue contre les actions extérieures.

L'étape de reproduction des bactéries est effectuée à partir d'un litre de bactéries mélangées dans 20 litres d'eau avec 5 kilos de poudre de lait enrichie en biocatalyseur et minéraux. Le mélange est laissé au repos pendant une durée de 24 heures à 37 degré Celsius. La multiplication des bactéries entraîne une baisse du pH qui passe de environ 5,7 à 4. L'utilisation de bactéries obtenues par prélèvement dans la nature et donc protégées génétiquement contre des infestations et des contaminations extérieures permet d'opérer dans des conditions non stérile durant les trois étapes du procédé.

Le mélange contenant les bactéries, obtenu après l'étape de reproduction, est ensuite mis en contact avec 25 kilos de poudre de lait enrichie en bio-catalyseur et minéraux, pour l'étape de tamponnage. Le mélange est laissé au repos pendant une durée de 24 heures à une température de 37 degré Celsius. On observe une première absorption d'eau qui permet de préparer les bactéries du mélange au choc osmotique de l'étape de stabilisation. On observe durant l'étape de tamponnage une baisse du pH qui passe d'environ 5,2 à 4,3.

Le produit obtenu après l'étape de tamponnage est ensuite mélangé avec des rafles de maïs extrudées pour l'étape de stabilisation, les proportions sont d'environ 600 kilos de rafles de maïs pour 100 kilos du produit obtenu après l'étape de tamponnage. Le mélange ainsi préparé est laissé au repos pendant une durée de 45 minutes à une température de 37 degré Celsius. Durant cette étape, on observe une augmentation du PH qui passe d'environ 4,3 à 5,3.

Le mélange de départ dans lequel les bactéries se sont reproduites contenait 70 % d'eau, le produit d'arrivée, obtenu après l'étape de stabilisation, ne contient plus que 12 % d'eau, il peut être conservé pendant une durée 3 mois dans un lieu sec à 37 degré Celsius.

Le produit obtenu par le procédé selon l'invention peut être incorporé directement dans l'alimentation des animaux pour lesquels il constitue un additif biologique d'efficacité zootechnique tout-à-fait intéressant. En effet, la symbiose microbienne, grâce à des géniteurs d'enzymes, tels que les cellulases et amylases, renforce le potentiel de l'animal pour une meilleure utilisation de la ration alimentaire; il est possible, ainsi, de favoriser la lactation, la croissance, la digestion ou l'appétit des animaux.

A titre d'exemple, chez les bovins l'utilisation d'un produit obtenu selon le procédé de l'invention, incorporé dans la ration alimentaire de l'animal à raison d' environ 17 à 50 grammes par jour, permet notamment une meilleure digestion de la ration, une croissance améliorée, une orientation métabolique vers la production de protéine, une amélioration de l'état sanitaire.

Le produit obtenu par le procédé de l'invention peut constituer également, selon la nature des bactéries utilisées, un additif biologique pour le traitement des lisiers; il permet entre autre d'améliorer l'homogénéité du lisier, une diminution de dégagement d'ammoniac, une réduction des odeurs.

Le procédé de l'invention est facile à mettre en oeuvre et ne nécessite pas un équipement couteûx et sophistiqué, le produit obtenu est facile d'emploi et trouve des applications diverses, il peut être stocké pendant une longue période sans nuire aux activités biologiques des micro-organismes qu'il contient.

## Revendications

1. Procédé de protection et de conservation de bactéries, caractérisé en ce qu'on effectue au moins les trois étapes successives suivantes dans des conditions non stériles :
- une première étape de reproduction de bactéries consistant en un mélange de bactéries issues de collections et de bactéries obtenues par prélèvement dans la nature,
- une deuxième étape de tamponnage par mise en contact des bactéries, après l'étape de reproduction, avec un milieu nutritif contenant de la poudre de lait enrichie en biocatalyseur et minéraux,
- une troisième étape de stabilisation par mise en contact du produit obtenu à la seconde étape avec un milieu contenant au moins un extrait de céréales.

2. Procédé de protection et de conservation de bactéries selon la revendication 1, caractérisé en ce que l'étape de reproduction est effectuée pendant une durée de 12 à 48 heures.

3. Procédé de protection et de conservation de bactéries selon la revendication 1 ou 2, caractérisé en ce que l'étape de tamponnage est effectuée pendant une durée de 18 à 24 heures.

4. Procédé de protection et de conservation de bactéries selon la revendication 1, caractérisé en ce que l'étape de stabilisation est réalisée par mise en contact du produit obtenu à la seconde étape avec des rafles de maïs broyées, extrudées ou floconnées.

5. Procédé de protection et de conservation de bactéries selon l'une quelconque des revendications 1 à 4, caractérisé en ce que l'étape de stabilisation est effectuée pendant une durée de 18 à 24 heures.

## Claims

1. A process for protection and conservation of bacteria, **characterised in that** at least the three following stages are carried out in succession under non-sterile conditions:
- a first stage of reproduction of bacteria consisting of a mixture of bacteria derived from collections and bacteria obtained from natural samples;
- a second stage of buffering by bringing the bacteria from the reproduction stage into contact with a nutritive medium containing milk powder enriched with a biocatalyst and minerals;
- a third stage of stabilisation by bringing the product obtained from the second stage into contact with a medium containing at least one cereal extract.

2. A process for protection and conservation of bacteria in accordance with claim 1, **characterised in that** the reproduction stage is carried out for a period of 12 to 48 hours.

3. A process for protection and conservation of bacteria in accordance with claim 1 or 2, **characterised in that** the buffering stage is carried out for a period of 18 to 24 hours.

4. A process for protection and conservation of bacteria in accordance with claim 1, **characterised in that** the stabilisation stage is carried out by bringing the product obtained in the second stage into contact with ground, extruded or flaked cobs of maize.

5. A process for protection and conservation of bacteria in accordance with any one of claims 1 to 4, **characterized in that** the stabilisation stage is carried out for a period of 18 to 24 hours.

## Patentansprüche

1. Verfahren zum Schützen und Konservieren von Bakterien, dadurch gekennzeichnet, dass es mindestens die drei folgenden Schritte umfasst, die nicht unter sterilen Bedingungen durchgeführt werden müssen:
- ein erster Schritt zur Vermehrung von Bakterien mit Hilfe eines Gemisches aus von Kolonien und in der Natur entnommenen Bakterien,
- ein zweiter Schritt zum Tamponieren dadurch, dass nach der Vermehrungsphase die Bakterien mit einem Nährmedium in Berührung gebracht werden, das mit Zellwirkstoffen und Mineralien angereichertes Milchpulver enthält,
- ein dritter Schritt zur Stabilisierung dadurch, dass das Endprodukt der zweiten Phase mit einem Medium in Berührung gebracht wird, das zumindest einen Getreideextrakt enthält.

2. Verfahren zum Schützen und Konservieren von Bakterien gemäss Anspruch 1, dadurch gekennzeichnet, dass die Vermehrung sich über einen Zeitraum von 12 bis 48 Stunden erstreckt.

3. Verfahren zum Schützen und Konservieren von Bakterien gemäss Anspruch 1 oder 2, dadurch gekennzeichnet, dass das Tamponieren sich über einen Zeitraum von 18 bis 24 Stunden erstreckt.

4. Verfahren zum Schützen und Konservieren von Bakterien gemäss Anspruch 1 dadurch, dass die Stabilisierung durch Vermischung des in der zweiten Phase erhaltenen Produktes mit gestossenen, extrudierten oder geflockten Maisspindeln erreicht wird.

5. Verfahren zum Schützen und Konservieren von Bakterien gemäss einem der Ansprüche 1 bis 4, dadurch gekennzeichnet, dass die Stabilisierung sich über einen Zeitraum von 18 bis 24 Stunden erstreckt.
